# EUROPÄISCHE PATENTSCHRIFT

(11) **EP 1 982 007 B1**
(45) Veröffentlichungstag und Bekanntmachung des Hinweises auf die Patenterteilung: **17.05.2017**
(21) Anmeldenummer: 07722775.9
(22) Anmeldetag: 27.01.2007
(51) Int. Cl.: C25D 11/02, C25D 11/26, C25F 3/02, C25F 3/08, A61L 27/04, A61L 27/06, A61L 27/56, A61C 8/00, A61C 13/00

(54) **VERFAHREN ZUR HERSTELLUNG EINES METALLKÖRPERS SOWIE METALLKÖRPER**
PROCESS FOR PRODUCING A METAL BODY AND METAL BODIES
PROCÉDÉ DE FABRICATION D'UN CORPS MÉTALLIQUE ET CORPS MÉTALLIQUE

(30) Priorität: 31.01.2006 DE 102006004653
(43) Veröffentlichungstag der Anmeldung: 22.10.2008
(73) Patentinhaber: Zipprich, Holger, 64283 Darmstadt (DE)
(72) Erfinder: Zipprich, Holger, 64283 Darmstadt (DE)
(74) Vertreter: Tergau & Walkenhorst Patentanwälte - Rechtsanwälte
(86) Internationale Anmeldenummer: PCT/EP2007/000711
(87) Internationale Veröffentlichungsnummer: WO 2007/088013

(56) Entgegenhaltungen:
- EP-A- 0 232 791
- EP-A- 1 159 935
- EP-A- 1 449 544
- WO-A-2004/008984
- WO-A-2004/098436
- WO-A-2006/072804
- WO-A-2006/104644
- CN-C- 1 147 625
- DE-A1- 10 230 720
- DE-A1- 19 643 555
- JP-A- 11 043 799
- RU-C1- 2 291 918
- US-A1- 2004 149 586
- US-A1- 2005 060 021
- US-A1- 2005 103 639
- US-B1- 6 290 834
- MACDONALD D E ET AL: "Thermal and chemical modification of titanium-aluminum-vanadium implant materials: effects on surface properties, glycoprotein adsorption, and MG63 cell attachment" BIOMATERIALS, ELSEVIER SCIENCE PUBLISHERS BV., BARKING, GB, Bd. 25, Nr. 16, Juli 2004 (2004-07), Seiten 3135-3146, XP004490245 ISSN: 0142-9612
- GORANSSON A ET AL: "Bone formation after 4 weeks around blood-plasma-modified titanium implants with varying surface topographies: an in vivo study" BIOMATERIALS, ELSEVIER SCIENCE PUBLISHERS BV., BARKING, GB, Bd. 24, Nr. 2, Januar 2003 (2003-01), Seiten 197-205, XP004390636 ISSN: 0142-9612
- ZHU X ET AL: "Effects of topography and composition of titanium surface oxides on osteoblast responses" BIOMATERIALS, ELSEVIER SCIENCE PUBLISHERS BV., BARKING, GB, Bd. 25, Nr. 18, August 2004 (2004-08), Seiten 4087-4103, XP004497071 ISSN: 0142-9612

## Beschreibung

Die Erfindung betrifft ein Verfahren zur Herstellung eines Metallkörpers, insbesondere zur Verwendung als Knochenimplantat, insbesondere als Dentalimplantat. Sie betrifft weiterhin einen nach dem Verfahren erhältliches Knochenimplantat.

Dentalimplantate sind in vielfältigen Formen bekannt. Sie werden meist durch Einschrauben an Stelle eines extrahierten oder ausgefallen Zahnes in den Kieferknochen eingesetzt, um dort nach einer Einheilphase von drei bis vier Monaten ein als Zahnersatz dienendes prothetisches Aufbauteil oder eine Krone zu halten. Dazu ist ein derartiges Zahnimplantat üblicherweise als geeignet geformter Metallkörper ausgebildet und in der Art eines Stiftes geformt und weist am apikalen Ende ein zumeist selbstschneidendes Schraubengewinde auf, mit welchem der Stift in das entsprechend präparierte Implantatbett eingesetzt wird.

In der Regel werden Dentalimplantate aus Titan, Zirkon, Niob oder Tantal oder aus gewebeverträglichen Legierungen, die eines dieser Elemente als Hauptbestandteil enthalten, hergestellt. Bei allen diesen Implantaten besteht das Ziel, dass die Knochensubstanz die Möglichkeit erhält, sich rasch und dauerhaft mit der Implantatoberfläche zu verbinden. Man spricht dabei auch von der so genannten Osseointegration. In diesem Zusammenhang ist bereits seit einiger Zeit bekannt, dass der mikroskopischen Struktur der Implantatoberflächen eine besondere Bedeutung zukommt. Insbesondere haben sich bislang poröse Oberflächen mit einer Porengröße im Mikrometerbereich als vorteilhaft erwiesen. Durch die vergrößerte Kontaktfläche zwischen Implantat und Knochen wird das Knochenwachstum gefördert und damit die Knochenanlagerungsrate nach dem postoperativen Trauma gesteigert.

Aus der EP 1 159 935 A1 sind überdies Implantate mit einer so genannten "Poreninnerhalb-Poren"-Struktur bekannt, bei denen zunächst durch Strahlenbehandlung vergleichsweise grobporige Oberflächenstrukturen erzeugt werden, in die anschließend feine Poren hineingeätzt werden, so dass sich Rauhigkeiten auf verschiedenen Längenskalen überlagern. Aus der DE 20 2005 002 450 U1 sind weiterhin Dentalimplantate mit einer homogenen nanostrukturierten Oberfläche bekannt. Derartige nanostrukturierte Oberflächen scheinen aufgrund eines besonders günstigen Benetzungsverhaltens das Einwachsen der Implantate und die Integration in die Knochensubstanz zu fördern.

Bislang gebräuchliche Methoden zur Oberflächenstrukturierung von Metallkörpern, insbesondere zur Verwendung als Dentalimplantate, umfassen das Sandstrahlen, das Ätzen, das elektrolytische Ätzen, die Laserbehandlung, die Funkenerosion, das Plasmaspritzen oder auch die Hochtemperaturelektrolyse. Sie sind entweder mit einem hohen Aufwand verbunden oder hinterlassen unerwünschte Verunreinigungen auf der Oberfläche. Weiterhin ist es mit Ausnahme der Lasertechnologie nach dem derzeitigen Wissensstand mit keinem der Verfahren mit vertretbarem Aufwand möglich den gesamten topographischen Bereich von 10 nm bis 500 µm auf eine Oberfläche zu übertragen. Das heißt, dass um Poren in Poren (siehe EP 1 159 935 A1) oder eine feine Struktur (z. B. Nanostruktur d. h. Strukturgröße kleiner 100 nm) einer gröberen Mikrostruktur (Strukturgröße ca. 1 µm bis 5 µm) zu überlagern ist es notwendig verschiedene Verfahren anzuwenden. Bei der vorliegenden Erfindung ist man in der Lage zuerst eine grobe Mikrostruktur (Strukturgröße größer 20 µm) zu erzeugen, diese mit einer feineren Mikrostruktur (Strukturgröße ca. 0,5 µm bis 20 µm) zu überlagern und beiden Strukturen noch eine Nanostruktur (Strukturgröße 10 nm bis 500 nm oder 10 nm bis 250 nm, vorzugsweise 10 nm bis 100 nm) zu überlagern. Insbesondere Nanostrukturen mit definierten geometrischen Parametern bzw. Oberflächentopographien auf Nanometerskala mit spezifischen (bio-)physikalischen oder chemischen Eigenschaften, die in der oralen Implantologie bei vertretbarem Herstellungsaufwand einen gesteigerten klinischen Nutzen gegenüber den herkömmlichen mikrostrukturierten Implantatoberflächen bieten, sind mit den bislang bekannten Methoden, wenn überhaupt, nur schwer zu fertigen. Zudem sind die bekannten Verfahren der Oberflächenstrukturierung häufig störanfällig gegenüber unvermeidbaren Störungen und Parameterschwankungen in der Prozessführung und führen somit häufig zu Artefakten und Produktionsausschuss.

Der Erfindung liegt daher die Aufgabe zugrunde, ein Verfahren zur Herstellung eines Metallkörpers anzugeben, mit dem auf besonders günstige und zuverlässige Weise eine Oberflächenvergrößerung erhältlich ist, bei welchem mittels der Prozessparameter (Ätzmedium/Elektrolyt, Spannungsverlauf, Stromdichte, Temperatur, Anwendungsdauer etc.) die Oberflächenbeschaffenheit bezüglich der mikroskopischen Rauhigkeiten und oder nanoskopische Poren/Strukturen gezielt erzeugt werden können, die besonders günstige Mikroretentionseigenschaften und oder besonders günstige Benetzungseigenschaften gewährleisten. Weiterhin soll ein Knochenimplantat mit in dem genannten Sinne besonders vorteilhaften Oberflächeneigenschaften angegeben werden, das insbesondere zum Einwachsen in den Knochen zur Verwendung als Dentalimplantat und oder als orthopädisches Implantat (z. B. Hüftgelenksimplantat) besonders geeignet ist.

Bezüglich des Verfahrens wird die Aufgabe erfindungsgemäß gelöst, mit den Merkmalen des Anspuchs 1.

Wie sich völlig überraschend und unerwarteterweise herausgestellt hat, führt gerade dieses Verfahren zur Ausbildung von Oberflächenstrukturen, die besonders günstige Benetzungseigenschaften gewährleisten und bei einer Verwendung des Metallkörpers als Dentalimplantat die Osseointegration, das heißt die Knochenanlagerung an der Implantatoberfläche, besonders gut stimulieren. Durch die Behandlung des Metall-Grundkörpers in der Art eines elektrolytischen Ätzens mit einem pulsierenden Elektrolysestrom bildet sich nämlich eine spezifische Nanostruktur auf der Oberfläche aus. Dabei sind eine Vielzahl vergleichsweise kleinerer Poren oder Vertiefungen mit einer mittleren Ausdehnung im Sub-Mikrometerbereich, vorzugsweise kleiner als 200 nm, anzutreffen. Derartige Strukturen können beispielsweise anhand elektronenmikroskopischer Aufnahmen nachgewiesen werden.

Es wird davon ausgegangen, dass die Beaufschlagung des Metall-Grundkörpers mit einem pulsierenden, also sich in einem vergleichsweise kurzen Zeitraum vergleichsweise stark änderndem Strom die bei einem Metallkörper eigentlich immer vorhandene oxidische Oberflächenschicht lokal und statistisch verteilt kurzzeitig aufbricht, so dass das Ätzmaterial an einzelnen Stellen vorübergehend in direktem Kontakt mit dem eigentlichen Metall treten kann. Über die im Elektrolysebad bereitgestellten Reaktionspartner, die derart gewählt sind, dass sie mit dem jeweiligen Metall-Material geeignete chemische Verbindungen eingehen, werden die herausgeätzten Metallionen gebunden und anschließend z. B. ausgefällt oder im Elektrolyten in Lösung gebracht und somit dauerhaft vom Metall-Grundkörper entfernt. Es wird vermutet, dass sich anschließend die aufgebrochene oxidische Oberflächenschicht des Metall-Grundkörpers wieder regeneriert, so dass der Ätzprozess wieder zum Erliegen kommt und somit lokale Krater mit nanoskopischen Dimensionen verbleiben. Beim nächsten Strompuls scheint dieser Prozess erneut einzusetzen, wobei dann wiederum statistisch verteilt an anderen Stellen des Metall-Grundkörpers die oxidische Oberflächenschicht lokal und vorübergehend zerstört wird.

Die vorgesehenen Reaktionspartner für das Metall des Grundkörpers, also die Ionen mit Bestandteilen aus der V. bis VII. Hauptgruppe des Periodensystems der Elemente, können dabei insbesondere als Salzbildner für das jeweilige Metall wirken. Insbesondere kann das Elektrolysebad Ionen umfassen, die aus den Elementen Stickstoff (N), Sauerstoff (O), Fluor (F), Chlor (Cl), Schwefel (S) und/oder Phosphor (P) bestehen oder diese als Bestandteile umfassen.

Gerade die bei diesem Prozess entstehenden Nanostrukturen scheinen im Allgemeinen das Benetzungsverhalten des Metallkörpers oder bei Verwendung als Dentalimplantat auch die Proteinanlagerung sowie die Collagen- und Zellbindung zu fördern. Dabei spielen insbesondere auch chemische Eigenschaften der Oberfläche im Mikrometerbereich und im Nanometerbereich eine wesentliche Rolle (z. B. hydrophil oder hydrophob, dotiert oder rein, etc.). Im vorliegenden Fall scheint ein für die orale Implantologie besonders wichtiger Vorteil der nach dem erfindungsgemäßen Verfahren hergestellten bzw. präparierten Metall- oder Implantatoberflächen insbesondere darin zu bestehen, dass diese einen ausgesprochen hydrophilen Charakter aufweisen, der beispielsweise auch nach längerem Kontakt des Metallkörpers mit der Erdatmosphäre nicht verloren geht. Als Maß für den hydrophilen Charakter dient insbesondere der Kontaktwinkel, den ein die Oberfläche benetzender Flüssigkeitstropfen mit der Oberfläche bildet. Wie sich gezeigt hat, führen die nach dem neuen Verfahren behandelten Metalloberflächen insbesondere bei Wasser zu einer ausgesprochen guten Benetzbarkeit mit Kontaktwinkeln von weniger als 10°. Das heißt, auf der Oberfläche befindlichte Flüssigkeitstropfen besitzen die Gestalt einer sehr flachen Kugeikappe. Der hierdurch zum Ausdruck kommende hydrophile Charakter der hergestellten Metallkörper bleibt zudem dauerhaft auch über einen Zeitraum von mehr als einigen Tagen erhalten.

Zweckmäßigerweise besteht der Metall-Grundkörper aus Titan oder aus einer titanhaltigen Legierung, insbesondere versetzt mit Chrom. Titan weist neben einer hohen Festigkeit eine hohe Korrosionsbeständigkeit und eine sehr gute Biokompatibilität auf. Daneben kommen aber prinzipiell auch noch andere Metalle oder Edelmetalle als Implantatwerkstoffe oder als Bestandteile davon in Betracht, z. B. Zirkon, Niob oder Vanadium. Vorteilhafterweise wird als Elektrolyt zur Bereitstellung der Salzbildner-Ionen Ionen mit Elementen aus der V-ten, VI-ten und/oder der VII-ten Hauptgruppe des Periodensystems, insbesondere eine Sulfationen (SO₄²⁻) und/oder Nitrationen (NO₃⁻) und/oder Nitritionen (NO₂⁻) und/oder Fluorionen (F⁻) und/oder Chloridionen (Cl⁻) und/oder Ionen der schwefligen Säure (SO₃²⁻⁾ und/oder Sulfidionen (S⁻) und/oder Phosphationen (Po₄³⁻), wobei diese Materialien gerade bei einem Metallkörper aus Titan besonders gut zur Bindung herausgeätzter Titan-Ionen geeignet sind. Weitere geeignete Salzbildner-Ionen können beispielsweise Chloridionen (Cl⁻) oder Phosphationen (PO₄³⁻) sein.

Vorteilhafterweise wird als Elektrolyt eine der Säuren Schwefelsäure (H₂SO₄), Salpetersäure (HNO₃), Salzsäure (HCl) ,salpetrige Säure (HNO₂), Phosphorsäure (H₃Po₄), schweflige Säure (H₂SO₃), Floursäure (HF), ein Gemisch aus mindestens zweien der genannten Säuren, oder eine wässrige Lösung mit Salzen der genannten Säuren oder ein Gemisch aus den Genannten verwendet. In einer alternativen Variante kann als Elektrolyt aber auch eine wässrige Natriumsulfatlösung oder eine Ammoniumsulfatlösung oder eine Natriumnitritlösung oder eine Ammoniumnitritlösung vorgesehen sein. In diesem Fall wird vorzugsweise eine Konzentration von ungefähr 5 g Natriumsulfat (Na₂SO₄) oder Ammoniumsulfat ((NH₄)₂SO₄) oder Natriumnitrit (NaNO₂) oder Ammoniumnitrit (NH₄NO₂) je 30 ml Wasser (H₂O) eingestellt. Es können aber auch andere Elektrolytlösungen zum Einsatz kommen, bei denen z. B. Sulfate, Sulfide, Nitrate, Nitride, Chloride, Fluoride oder Phosphate in einer wässrigen oder nicht wässrigen Flüssigkeit gelöst sind. Die Elektrolyttemperatur bei der Durchführung des Verfahrens sollte oberhalb von etwa 0 °C und unterhalb der Siedetemperatur des Elektrolyts gewählt werden und beträgt vorteilhafterweise 40 °C bis 120 °C, insbesondere etwa 50 °C.

Eine weitere Möglichkeit ist Salze oder ein Gemisch aus mehreren Salzen zu schmelzen und als Ätzmedium/Elektrolyt zu verwenden. Hierbei bestehen die Möglichkeiten der Schmelze von Hxdratgebundenen Salzen (z. B. Calcium) welche sich in ihrem eigenen Kristallwasser lösen oder geschmolzene, wasserfreie Salze. Eine Variante ist die Schmelzen von Calciumchlorid Hexahydrat bei Temperaturen größer 30,2 °C. Ein weiteres Beispiel ist die Beaufschlagung einer Hochtemperaturelektrolyse mit einem Wechselstrom mit oder ohne Gleichstromanteil.

Vorteilhafterweise ist der zeitlich pulsierende bzw. zeitlich veränderliche Elektrolysestrom ein Wechselstrom, ändert also periodisch seine Richtung. Dem Wechselstrom ist dabei vorzugsweise ein Gleichstromanteil derart überlagert, dass der Metall-Grundkörper ausschließlich als Anode und nicht als Kathode wirksam ist. Besonders vorteilhaft im Hinblick auf die angestrebten Oberflächenstrukturen und deren mikrobiologische, chemische und physikalische Eigenschaften ist ein Rechteck-Wechselstrom, vorzugsweise mit entsprechendem Gleichstromanteil.

Weiterhin ist es vorteilhaft, wenn an die Elektroden des Elektrolysebades eine Wechselspannung mit einer Frequenz von vorzugsweise 1 Hz, insbesondere größer 1 Hz, angelegt wird, deren Amplitude in aufeinanderfolgenden Zeitintervallen schrittweise, vorzugsweise in Schritten von ungefähr 5 V oder weniger, von ungefähr 5 V bis auf ungefähr 30 V gesteigert wird. Die wechselspannungsbedingte Reduzierung der Spannung, eine vergleichsweise langsame Steigerung der Spannung bezüglich der Schrittweite und eine längere Verweildauer auf dem jeweiligen Spannungsniveau verhindern dabei einen unkontrollierten Materialabtrag von der Implantatoberfläche. Wie sich herausgestellt hat, stellt eine Intervalllänge von ca. 5 Minuten einen besonders günstigen Kompromiss dar hinsichtlich einer zuverlässigen und zielgerichteten Verfahrensführung einerseits und einer unter ökonomischen Gesichtspunkten vertretbaren, nicht zu langen Gesamtbehandlungsdauer andererseits.

Andererseits hat sich überraschenderweise aber auch herausgestellt, dass sich besonders günstige Behandlungsergebnisse erreichen lassen, indem in den Phasen der Spannungsänderung, also den so genannten Transientenphasen, eine besonders hohe Änderungsgeschwindigkeit der Spannung von mindestens 1 V/s, vorzugsweise größer als 10 V/s, insbesondere größer als 1000 V/s, gewählt wird. Die besten Ergebnisse wurden mit den Spannungsänderungsgeschwindigkeiten eines Rechtecksignals erreicht. Damit lassen sich vergleichsweise intensive, pulsartige Spannungsänderungen in positiver und negativer Richtung erreichen, die offenbar die gewünschte nanoskopische Ausformung von Oberflächenstrukturen besonders begünstigen.

In besonders vorteilhafter Ausgestaltung wird mit dem Verfahren ein Knochenimplantat für medizinische Zwecke, insbesondere zum Einsatz oder zur Inseration im menschlichen Körper, vorzugsweise ein Dentalimplantat oder Hüftimplantat, hergestellt. Dazu wird vorzugsweise als Metall-Grundkörper ein mit einer mikrostrukturierten, vorzugsweise elektrisch oder elektrochemisch hergestellten Oberfläche versehener Implantat-Grundkörper verwendet. Die Implantatoberfläche des solchermaßen hergestellten Dentalimplantats weist einerseits eine Vielzahl von zwar unregelmäßig angeordneten, aber statistisch gesehen annähernd homogen verteilten Mikroporen oder "Kratern" mit einer mittleren Ausdehnung von ca. 0,5 µm bis 100 µm, vorzugsweise 0,5 µm bis 20 µm auf, wobei andererseits innerhalb dieser Mikroporen jeweils die durch das gepulste Ätzen erzeugten Nanoporen angeordnet sind. Derartig ausgestaltete Dentalimplantante unterstützen und beschleunigen durch Anregung der Aktivität der knochenbildenden Zellen, der Osteoblasten, den nach der Implantation stattfindenden Heilungsprozess nachdrücklich. Dabei ist das Herstellungsverfahren für das Implantat auch im industriellen Maßstab vergleichsweise einfach und kostengünstig durchzuführen und zu kontrollieren. Die Prozessparameter werden dabei vorzugsweise derart gewählt, dass sich auf der Oberfläche des Implantat-Grundkörpers eine der Mikrostruktur überlagerte Nanostruktur der oben beschriebenen Art ausbildet.

Zu Beginn des auf die Erzeugung der Nanostruktur ausgerichteten elektrolytischen Ätzens sollte der Implantat-Grundkörper vorzugsweise bereits mit einer mikroskopischen Oberflächenstruktur versehen und/oder chemisch aktiviert sein. Dieser Vorbehandlung kommt im Hinblick auf die erzielbaren Endresultate eine wichtige Rolle zu. Zwei der folgenden sechs Vorbehandlungsmethoden haben sich als besonders wirkungsvoll erwiesen:

### Vorbehandlungsmethoden:

1. In einer ersten vorteilhaften Variante wird das erfundene Verfahren selbst zur primären Strukturierung der Oberfläche eingesetzt. Die Mikrostruktur wird auf der Oberfläche des Implantat-Grundkörpers in einem der Nanostrukturierung vorgelagerten Verfahrensschritt dadurch erzeugt, dass der Implantat-Grundkörper zuerst in einem Elektrolysebad mit einem pulsierenden Strom beaufschlagt wird, wobei als Elektrolyt eine Chloridionen (Cl⁻) enthaltende wässrige Lösung oder Salzsäure als Hauptbestandteil beinhaltet verwendet wird, und wobei der Implantat-Grundkörper anschließend in ein Säurebad, vorzugsweise ein Schwefelsäurebad, eingetaucht wird. Dabei bildet sich auf dem Implantat-Grundkörper zuerst eine aus den elektrolytischen Reaktionsprodukten bestehende Schicht, insbesondere aus Titan-Chlor-Verbindungen. Diese wird anschließend durch Eintauchen des Implantats in ein Säurebad, vorzugsweise ein Schwefelsäurebad, entfernt.
2. In einer alternativen zweiten Variante wird die Mikrostruktur in einem vorgelagerten Verfahren geätzt werden, vorzugsweise elektrolytisch. Als Grundätzmedium/-elektrolyt können die Säuren Salzsäure, Phosphorsäure, Fluorsäure, Schwefelsäure oder Salpetersäure oder ein Gemisch aus mindestens zweien der Säure verwendet werden.
3. Eine weitere Vorbehandlungsmethode ist das ätzen oder elektrolytische ätzen in geschmolzenen Salzen, welche sich in Ihrem eigenen Kristallwasser gelöst haben, vorzugsweise geschmolzene, wasserfreie Salze. Im speziellen kann dies als Hochtemperaturelektrolyse (HTE) ausgeführt sein.
4. Eine weitere Vorbehandlungsmethode ist das, chemische, elektrolytische, insbesondere in Ausführung als Hochtemperaturelektrolyseverfahren, Beschichten mit Titan, Zirkon, Tantal, Niob, Chrom, Eisen oder einer Legierung mit einem der genannten Elemente als Hauptbestandteil.
5. Als weitere Vorbehandlungsverfahren können das Sandstrahlen, das Funkenerodieren, die Laserbehandlung, das Plasmaspritzen oder andere Abtragende Oberflächenverfahren aufgeführt werden.
6. Weiterhin führt die Kombination einer oder mehrerer der genannten Vorbehandlungsmethoden zu einer Variation der Oberflächentopographie.

Bezüglich des Knochenimplantats wird die genannte Aufgabe gelöst mit den Merkmalen des Anspruchs 9. In besonders vorteilhafter Weise wird das genannte Verfahren zur Herstellung eines derartigen Knochenimplantats verwendet. Wie sich nämlich herausgestellt hat, kann als Ergebnis des elektrolytisch gepulsten Ätzens eine vornehmlich durch Poren in der Oberfläche oder aber alternativ auch eine vornehmlich durch Erhebungen auf der Oberfläche geprägte Struktur entstehen. Die lateralen Dimensionierungen sollten dabei in jedem Fall im nanoskopischen Bereich liegen, so dass die gewünschten Oberflächeneigenschaften hinsichtlich Hydrophilie oder auch hinsichtlich des Knochenwachstums erreicht werden. Im Falle der Ausbildung von Oberflächenerhebungen sollten diese daher ebenfalls im Mittel laterale Ausdehnungen von nicht mehr als etwa 250 nm aufweisen.

Das Knochenimplantat ist dabei besonders vorteilhaft als Dentalimplantat ausgestaltet. Es ist vorzugsweise aus Titan oder aus einer titanhaltigen Legierung, vorteilhafterweise mit einer mikrostrukturierten Oberfläche, gebildet, wobei der Mikrostruktur eine Nanostruktur überlagert ist, und wobei im Bereich der Oberfläche vorzugsweise Stickstoffatome und/oder Stickstoffverbindungen angelagert oder eingeschlossen sind. Dem liegt die Überlegung zugrunde, dass sich durch das gezielte Einbringen oder Aufbringen von Stickstoffatomen in den Atom- oder Molekülverbund der Implantatoberfläche in der Art einer Stickstoffdotierung ein besonders positiver Einfluss auf die Knochenanlagerung erreichen lässt.

Bezüglich der Oberflächenvergrößerung bei Metallkörpern wird erfindungsgemäß dieser Metallkörper aus insbesondere Titan, Chrom, Eisen, Zirkon, Tantal, Niob oder aus einer Legierung mit mindestens einem dieser Metalle als Hauptbestandteil, in einem Umgebungsmedium vorzugsweise in einem flüssigen Elektrolyt, welcher Ionen beinhaltet, wovon mindestens eines aus einem Element der V. bis VII. Hauptgruppe des Periodensystems der Elemente besteht oder gebildet wird oder ein derartiges Element als Bestandteil umfasst, und während der Anwendung mit einer zeitlich veränderlichen Spannung beaufschlagt, welche mit Spannungsänderungen von mindestens 1 V/s, 10 V/s, vorzugsweise größer als 100 V/s beaufschlagt wird.

Vorzugsweise ist dabei die angelegte Spannung mindestens 1 V, vorzugsweise größer 5 V.

In weiterer vorteilhaften Ausgestaltung ist das beim Verfahren eingesetzte Medium ein wasserhaltiger Elektrolyt, der Ionen mit mindestens einem der Elemente aus der fünften, sechsten oder siebten Hauptgruppe beinhaltet und/oder das beim Verfahren eingesetzte Medium mindestens eine Säure mit einem Element aus der fünften, sechsten oder siebten Hauptgruppe und/oder das beim Verfahren eingesetzte Medium teilweise, vorzugsweise gänzlich aus mindestens einem geschmolzenen Salz oder einer chemischen Verbindung, welches mindestens aus einem der Elemente aus der fünften, sechsten oder siebten Hauptgruppe besteht.

Weiterhin beinhaltet in vorteilhafter Ausführung das Umgebungsmedium zur Strukturierung der Oberfläche im Bereich von 0,25 µm bis 500 µm als Bestandteil auch Chlor, Chlorionen und/oder eine chlorhaltige Säure, vorzugsweise Salzsäure und/oder zur Strukturierung der Oberfläche im Bereich von 1 nm bis 250 nm als Bestandteil auch Schwefel, Schwefellonen und/oder eine schwefelhaltige Säure, vorzugsweise Schwefelsäure und/oder zur Strukturierung der Oberfläche im Bereich von 0,25 µm bis 100 µm als Bestandteil auch Stickstoff, Stickstoffionen und/oder eine stickstoffhaltige Säure, vorzugsweise Salpetersäure und/oder zur Strukturierung der Oberfläche im Bereich von 1 nm bis 250 nm als Bestandteil auch Stickstoff, Stickstoffionen und oder eine stickstoffhaltige Säure, vorzugsweise Salpetersäure und/oder zur Strukturierung der Oberfläche im Bereich von 1 nm bis 250 nm als Bestandteil auch Phosphor, Phosphorionen und/oder eine phosphorhaltige Säure, vorzugsweise Phosphorsäure und/oder zur Strukturierung der Oberfläche im Bereich von 1 nm bis 250 nm als Bestandteil auch Fluor, Fluorionen und/oder eine fluorhaltige Säure, vorzugsweise eine schwache Fluorsäure.

Die mit der Erfindung erzielten Vorteile bestehen insbesondere darin, dass mittels eines einfach und kostengünstig zu realisierenden elektrochemischen Verfahrens ein Metallkörper, insbesondere zur Verwendung als Dentalimplantat, mit einer Nanostruktur und einer Nanorauhigkeit fertigbar ist, welche sich vorteilhaft auf den Heilungsprozess nach der Verankerung des Implantats im Kieferknochen und insbesondere auch auf die erreichbare Festigkeit der Knochen-Implantat-Verbindung auswirkt. Durch die Dotierung von Fremdatomen, insbesondere Stickstoffatomen, in die Implantatoberfläche lässt sich der Effekt noch verstärken. Darüber hinaus lassen sich aufgrund der Nano-Oberflächenstruktur des Metallkörpers im Hinblick auf die damit verbundenen hydrophilen Eigenschaften und/oder Kapillarwirkungen Flüssigkeiten besonders einfach und wirksam in die Oberfläche einbringen. Dies könnte beispielsweise dazu verwendet werden, Medikamente oder sonstige Wirkstoffe oder Reagenzien an der Oberfläche zu platzieren. Aufgrund der guten Benetzbarkeit sind aber auch andere vorteilhafte Anwendungen denkbar, wobei beispielsweise die Aufbringung von Lacken, Klebern oder sonstigen Oberflächenbeschichtungen auf den Metallkörper deutlich erleichtert ist.

Das durch die erhaltene Nanostruktur oder Nanorauigkeit erhaltene besonders günstige Hydrophilverhalten der behandelten Oberfläche lässt sich beispielhaft durch den dabei erhaltenen charakteristischen Benetzungswinkel erkennen, der insbesondere kleiner als 15 % ist. Das damit erhaltene Hydrophilverhalten hält zudem durch die Nanoporen, Nanostrukturen, die Dotierung oder Anlagerung von Stickstoffatomen/-verbindungen auf und/oder an der Oberfläche vergleichsweise länger an als bei einer Metalloberfläche, die chemisch oder elektrochemisch aktiviert wurde.

Ausführungsbeispiele der Erfindung werden anhand einer Zeichnung näher erläutert. Darin zeigen:
- Fig. 1.: ein Dentalimplantat in einer teilweise geschnittenen Seitenansicht,
- Fig. 2: schematisch eine Vorrichtung zur elektrolytischen Behandlung eines Dentalimplantats, und
- Fig. 3 bis Fig. 37: eine Anzahl von elektronenmikroskopischen Aufnahmen der durch die elektrolytische Behandlung erzeugten Implantatoberflächen.

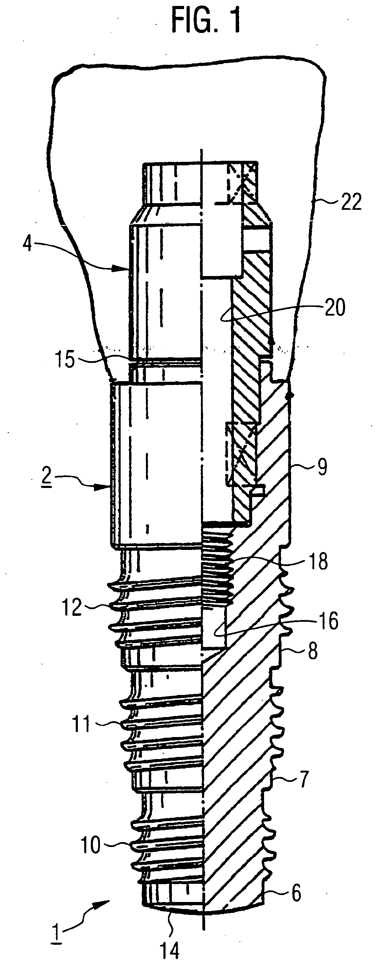

Fig. 1 zeigt teilweise in einer Ansicht und teilweise in einem axialen Schnitt ein zweiteiliges Dentalimplantat 1 mit einem Pfostenteil 2 und mit einem Aufbauteil 4. Das Pfostenteil 2 und ebenso das Kopf- oder Aufbauteil 4 bestehen aus Metall, und zwar insbesondere aus Titan oder einer Titanlegierung. Das Pfostenteil 2 ist als Stufenschraube ausgebildet und enthält drei Stufen 6 bis 8, welche jeweils ein selbstschneidendes Gewinde 10 bis 12 mit gleicher Steigerung aufweisen. Die dem apikalen Ende 14 nächstliegende Stufe 6 besitzt den kleinsten Durchmesser. Die dem Aufbauteil 4 nächstliegende Stufe 9 weist hingegen eine glatte zylinderische Außenfläche auf. Das Pfostenteil 2 besitzt am koronalen Ende 15 eine Innenbohrung 16, in welche das Kopfoder Aufbauteil 4 eingesetzt ist, und welche ferner ein Innengewinde 18 enthält. Mit einer hier nicht weiter dargestellten Schraube, welche durch eine Durchgangsbohrung 20 des Aufbauteils 4 hindurch geführt und in das Innengewinde 18 eingeschraubt ist, erfolgt die Verbindung des Aufbauteils 4 mit dem Pfostenteil 2. Mit dem Aufbauteil 4 kann in bekannter Weise eine Krone 22 oder dergleichen verbunden werden.

Das Pfostenteil 2 wird in einem entsprechend aufbereiteten Implantatbett des Kieferknochens verankert. Die Gewindekonstruktion gewährleistet dabei eine hohe Primärstabilität und eine gleichmäßige Weiterleitung der bei Kaubelastung auftretenden Kräfte in den Kieferknochen. Darüber hinaus sollte der Knochen während der sich an die Implantation anschließenden Heilungsphase möglichst direkt an das Implantat anwachsen und sich mit ihm innig verbinden. Dieser Prozess, die so genannte Osseointegration, wird durch eine Anrauhung der Implantatoberfläche deutlich verbessert. Im Prozess der Anrauhung und der Oberflächenstrukturierung unterscheiden sich viele am Markt angebotene Implantate, wobei sich bislang noch keine bestimmte Art der Oberflächenbearbeitung wissenschaftlich gesehen oder kommerziell durchsetzen konnte. Es besteht im Gegenteil das Bedürfnis, neuartige Verfahren zu entwickeln, die zu verbesserten Oberflächeneigenschaften führen und dabei insbesondere die Erkenntnisse der Nanostrukturforschung berücksichtigen, umsetzen und weiterentwickeln.

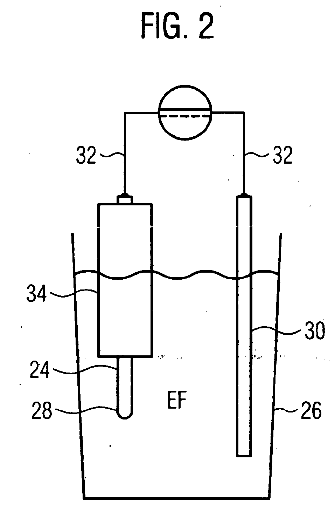

Im vorliegenden Fall kommen im Wesentlichen elektrolytische Ätzverfahren zum Einsatz, die auf einer Vorbehandlung eines Implantat-Rohlings zur Nanostrukturierung aufbauen, so dass sich insgesamt eine mikrostrukturierte Oberfläche mit eingelagerten Nanoporen ausbildet.. Die beiden Verfahrensschritte werden jeweils in einer Elektrolysevorrichtung, wie in Fig. 2 schematisch dargestellt, durchgeführt. Der zu behandelnde Implantat-Grundkörper oder Titanrohling 24 taucht dabei zumindest teilweise in eine in einem Behälter 26 gelagerte Elektrolytflüssigkeit EF ein und bildet eine Elektrode 28. Von dieser ersten Elektrode 28 beabstandet taucht eine zweite Elektrode 30, im Ausführungsbeispiel aus Titan, Platin oder Gold gefertigt, in die Elektrolytflüssigkeit EF ein. Die beiden Elektroden 28, 30 sind über elektrisch leitende Drahtverbindungen 32 an eine regelbare Spannungsquelle, die je nach Bedarf eine zeitlich konstante oder ein zeitlich variierende elektrische Spannung liefert, angeschlossen. Im Ausführungsbeispiel wird der Implantat-Grundkörper 24 von einem elektrisch isolierenden Halteelement 34 mechanisch gehalten, wobei zur elektrischen Kontaktierung ein elektrisch leitendes Innenelement (in Fig. 2 nicht sichtbar) durch das Halteelement 34 hindurchgeführt ist. Infolge der in der Elektrolytflüssigkeit EF gelösten, von Elektrode zu Elektrode wandernden Ionen, die als Ladungsträger für den elektrischen Stromtransport fungieren, wird der Stromkreis geschlossen. Die Metallionen oder Elektroden werden dabei an der Kathode durch Zugabe von Elektronen zu reinem Metall reduziert. Das Anodenmaterial wird unter Abgabe von Elektronen zu Metallionen oxidiert. Über eine hier nicht dargestellte regelbare Heizeinrichtung lässt sich die Temperatur der Elektrolytflüssigkeit EF einstellen.

### I. Mikrostrukturierung der Implantatoberfläche

Als Elektrolyt wird eine wässrige Lösung aus 30 ml Wasser (H₂O) und 5 g Natriumchlorid (NaCl) oder aus 30 ml Wasser (H₂O und 5 g Ammoniumchlorid (NH₄CL) gewählt. Alternativ können auch andere Salze, die beim Lösen in Wasser Chloridionen (Cl⁻) freisetzen, oder Salzsäure (HCL) zur Anwendung kommen. Die Elektrolyse wird bei einer Elektrolyttemperatur von 50 °C bis 60 °C durchgeführt, wobei das Titanimplantat als Anode wirksam ist (so genannte anodische Bestromung). Zwischen der Anode und der Kathode liegt ein zwischen 0 V oder geringer und einem Maximalwert pulsierendes, rechteckförmiges Spannungssignal mit einer Frequenz von 1 Herz an. Der Maximalwert, also die Amplitude des Rechtecksignals wird in aufeinanderfolgenden Zeitintervallen von jeweils 5 Minuten in 5-V-Schritten von 5 V bis auf 30 V gesteigert. Durch das Pulsieren der Spannung und die langsame Steigerung der Amplitude wird eine unkontrollierte Reaktion vermieden, die ansonsten dazu führen könnte, dass es an einigen Bereichen des Implantats zu einem unkontrollierten Materialabtrag kommt, während an anderen Bereichen die Reaktion ausbleibt.

Die bei der Elektrolyse als Reaktionsprodukte anfallenden Titan-Chlor-Verbindungen. sind im Elektrolyten nicht löslich. Die Reaktionsprodukte wachsen auf der Oberfläche des Dentalimplantats zum einen nach außen und greifen diese zum anderen unter Ausbildung von Vertiefungen an (Ätzwirkung). Dieser Prozess findet an der Oberfläche relativ ungleichmäßig statt.

Anschließend werden die Reaktionsprodukte durch Eintauchen des Implantats in ein temperiertes (z. B. 60 °C) Schwefelsäurebad entfernt. In diesem Fall ist eine Verweildauer von rund 30 bis 60 Minuten im Schwefelsäurebad zweckmäßig und ausreichend. Nach der auf diese Weise bewerkstelligten Entfernung der Schicht aus Reaktionsprodukten weist die Oberfläche des Dentalimplantats eine Rauhigkeit mit einer Strukturgröße im Bereich von 20 µm bis 100 µm auf, der vereinzelt Strukturen mit einer Größe von weniger als 1 µm oder mehr als 100 µm überlagert sein können. Durch die Variation der Zeitintervalle und der Steigerungsrate (in Volt pro Schritt) während der dem Schwefelsäurebad vorangehenden Elektrolysephase kann die Rauhigkeit variiert werden.

### II. Nanostrukturierung der Implantatoberfläche

### a) Variante 1

Der nach einem der oben beschriebenen Verfahren vorbehandelte und daher bereits mikrostrukturierte Implantat-Grundkörper wird in eine wässrige Elektrolytlösung getaucht, bei der 5 g Natriumsulfat (NaSO₄) oder 5 g Ammoniumsulfat ((NH₄)₂SO₄) in 30 ml Wasser gelöst sind, und dann bei einer Elektroyttemperatur von 50 °C mit einem rechteckförmigen Wechselstrom mit einer Frequenz von 1 Hz beaufschlagt. Die dadurch im Elektrolysebad vorgehaltenen Sulfationen (SO₄²⁻) dienen als Salzbildner und somit als geeignete Reaktionspartner für das metallische Titan. Alternativ kann beispielsweise auch eine 98-prozentige Schwefelsäure oder eine 60-prozentige Phosphorsäure als Elektrolyt verwendet werden. An den Elektroden 28, 30 der Elektrolysevorrichtung, das heißt am Dentalimplantat 24 und an der dazu korrespondierenden zweiten Elektrode 30, liegt eine rechteckförmige Wechselspannung mit einer anfänglichen Amplitude von 5 V an, welche in 5-V-Schritten langsam bis auf mindestens 10 V, vorzugsweise 60 V oder größer 60 V, gesteigert wird. Die Verweildauer im jeweiligen Zeitintervall beträgt 5 Minuten, so dass sich eine Gesamtbehandlungsdauer von etwa einer halben Stunde ergibt.

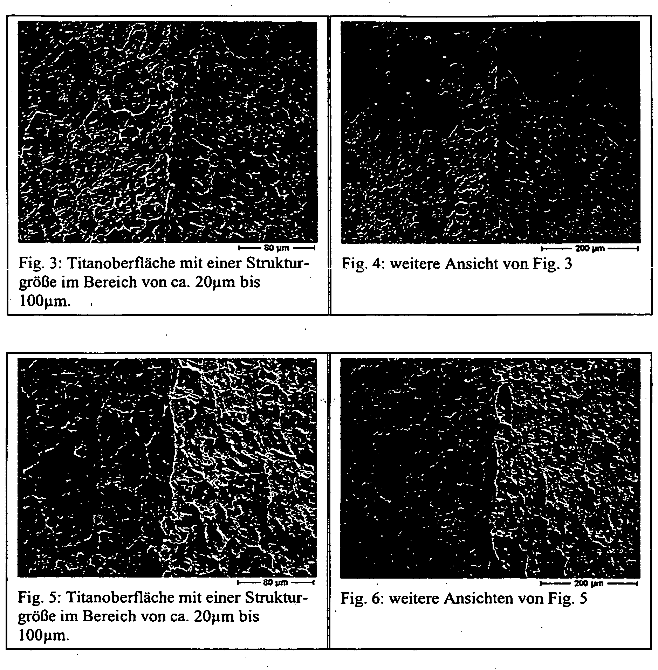

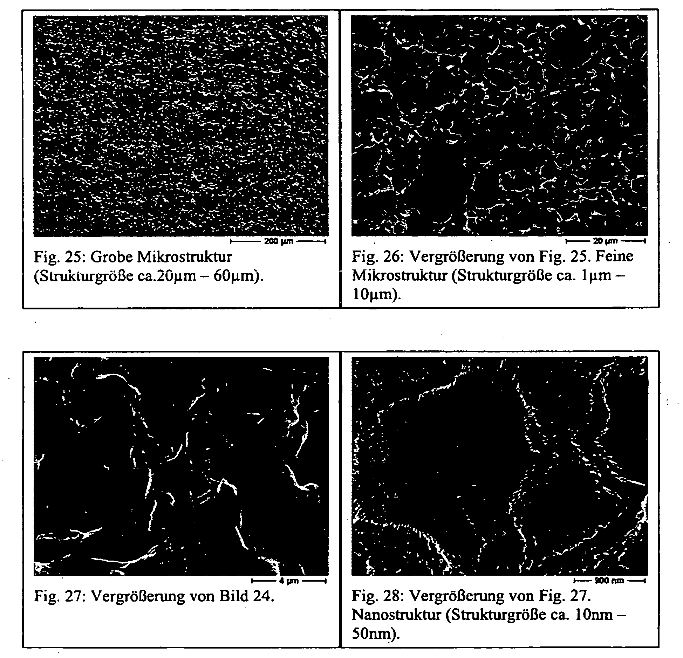

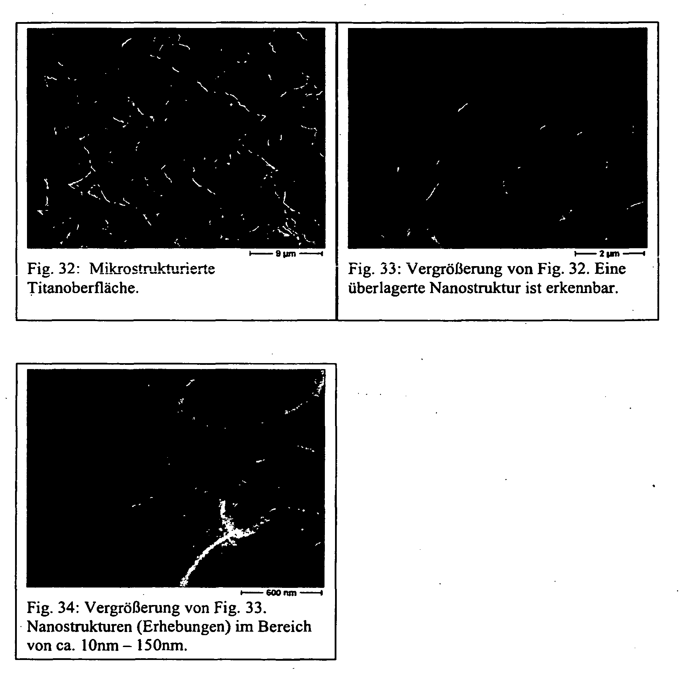

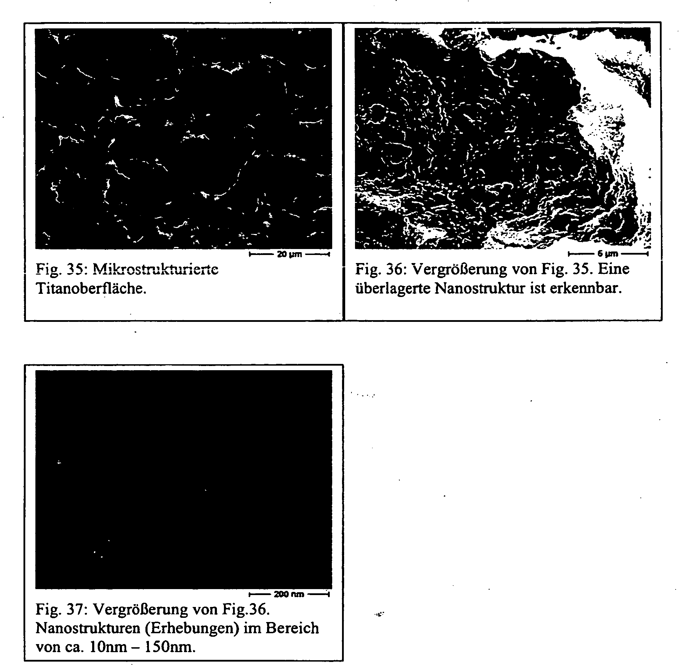

Bei der Behandlung bildet sich auf dem Implantat eine Titanoxidschicht, die die anfänglich bereits vorhandenen Mikrostrukturen gleichmäßig überzieht. Daneben bildet sich eine der Mikrostruktur aufgeprägte oder überlagerte Nanostruktur aus, deren Poren eine mittlere Ausdehnung von weniger als ein Mikrometer, je nach den eingestellten Verfahrensparametern üblicherweise 10 nm bis 900 nm, besitzen. Einige Beispiele für die derart hergestellten Oberflächenstrukturen sind in den elektronenmikroskopischen Aufnahmen der Fig. 3 bis Fig. 37, jeweils bei verschiedenen Vergrößerungsfaktoren, gezeigt. Der Maßstab ist jeweils innerhalb der Figuren angegeben. Die Oberflächen sind stark hydrophil, was bei Kontakt mit Flüssigkeiten zu einer besonders innigen Benetzung führt.

### b) Variante 2

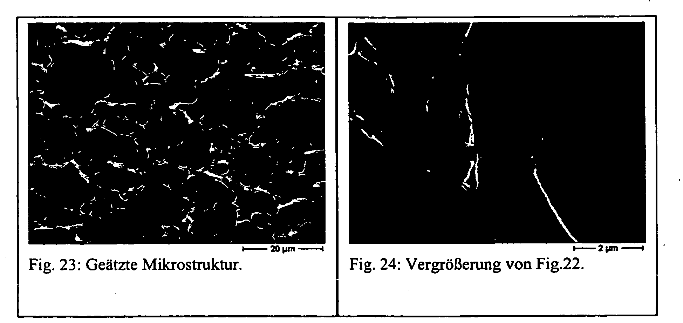

Variante 2 kann sowohl mit als auch ohne eine der sechs genannten Vorbehandlungsmethoden angewandt werden. Als Elektrolyt ist eine Lösung von 5 g Natriumnitrit (NaNO2) in 30 ml Wasser oder 5 g Ammoniumnitrit in 30 ml Wasser vorgesehen. Alternativ kann auch ein eine 60-prozentige Salpetersäure verwendet werden. Ansonsten entsprechen die Einzelheiten der Verfahrensführung und die Parameter den oben unter II a) genannten. Hierbei kann eine Überlagerung von drei Strukturen (siehe Fig. 23 bis 26) entstehen. Das heißt, einer groben Rauhigkeit von ca. 20 µm bis 60 µm ist eine feine Mikrostruktur mit einer Rauhigkeit von ca.1 µm bis 10 µm überlagert und dieser noch eine Nanostruktur mit einer Strukturgröße von ca. 10 nm bis 50 nm. Darüber hinaus können sich auf der Oberfläche elementarer Stickstoff oder Stickstoffver-bindungen an- oder einlagern.

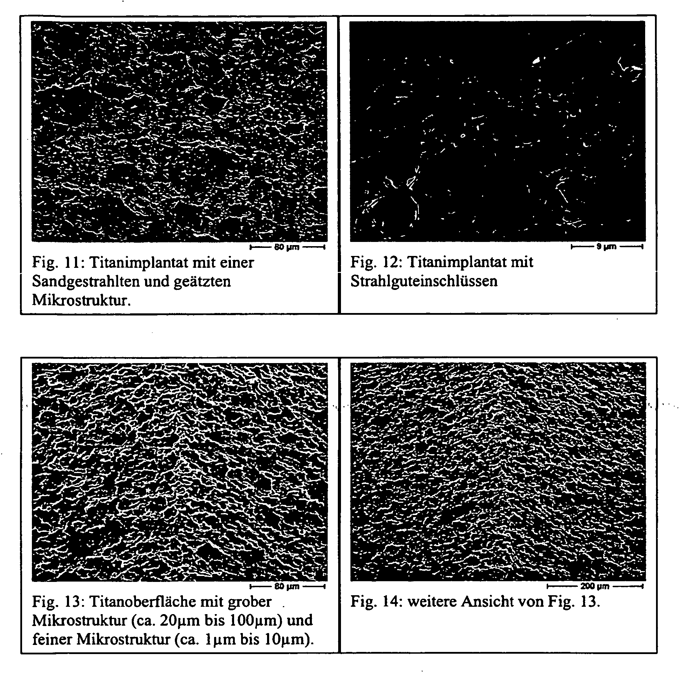

Fig. 3 bis 8 zeigen drei Möglichkeiten der ersten Vorbehandlungsmethode. Dieser Verfahrensschritt kann das bei anderen Ätzverfahren vorgelagerte Sandstrahlen (Vergleich mit einer am Markt befindlichen Oberfläche Fig. 11) bei gleicher oder ähnlicher Oberflächentopographie ersetzten und auf diesem Wege unerwünschte Einschlüsse des Strahlguts (Fig. 12) verhindern. Eingeschlossenes Strahlgut hat einen negativen Einfluss auf die Knochenanlagerung auf dem Implantat.

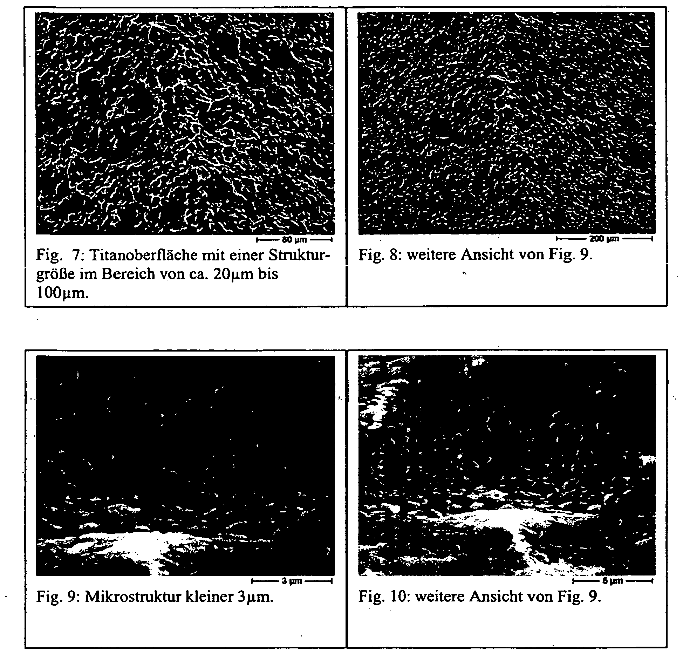

Fig. 7 bis 10 zeigen die Überlagerung einer groben Mikrostruktur (Strukturgröße 20 µm bis 80 µm) und einer feinen Mikrostruktur (Strukturgröße kleiner 1 µm) als Produkt der Vorbehandlungsmethode 1.

Die Fig. 13 und 14 zeigen die Überlagerung einer groben Mikrostruktur aus der Vorbehandlungsmethode 1 und einer feinen Mikrostruktur aus der Vorbehandlungsmethode 2.

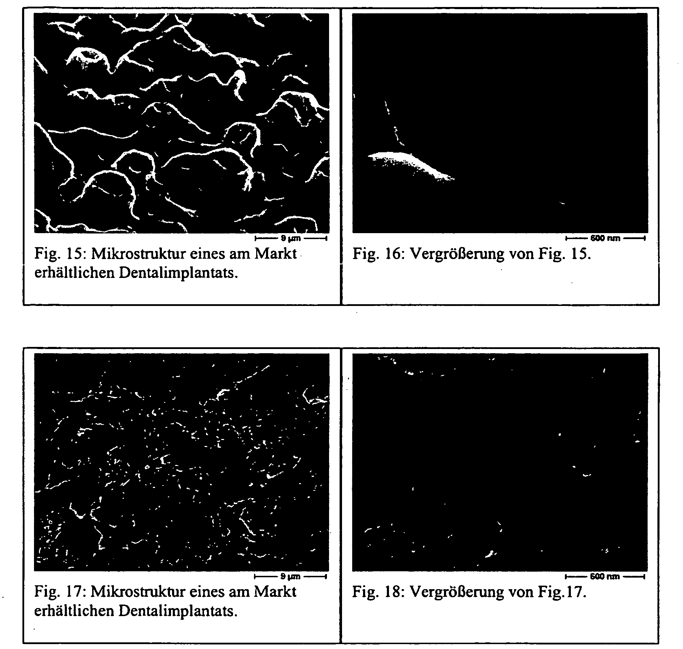

Die Fig. 15 bis 22 zeigen die Mikrostrukturen von vier am Markt erhältlichen Implantaten. Die Oberflächen wurden in einem letzten Verfahrensschritt geätzt oder mit Titan beschichtet. Die jeweils nächste Figur zeigt eine Ansicht, bei welcher eine Nanostruktur erkennbar sein müsste. Keines der vier am Markt erhältlichen Implantatoberflächen zeigt eine nennenswerte Nanostruktur, welche die Benetzungseigenschaften beeinflussen könnte.

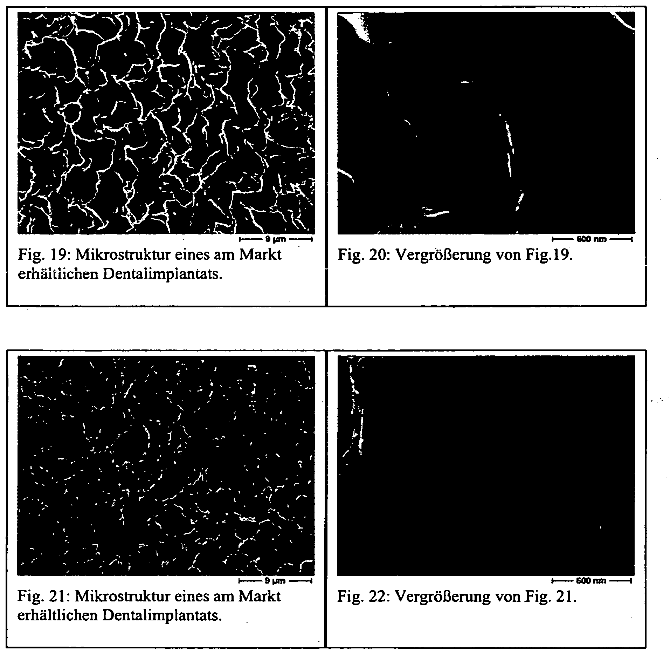

Die Fig. 23 und 24 zeigen eine Titanoberfläche, welche nach dem Vorbehandlungsverfahren 2 geätzt wurde. In Fig. 22 ist zu erkennen, dass keine nennenswerte Nanostruktur vorhanden ist.

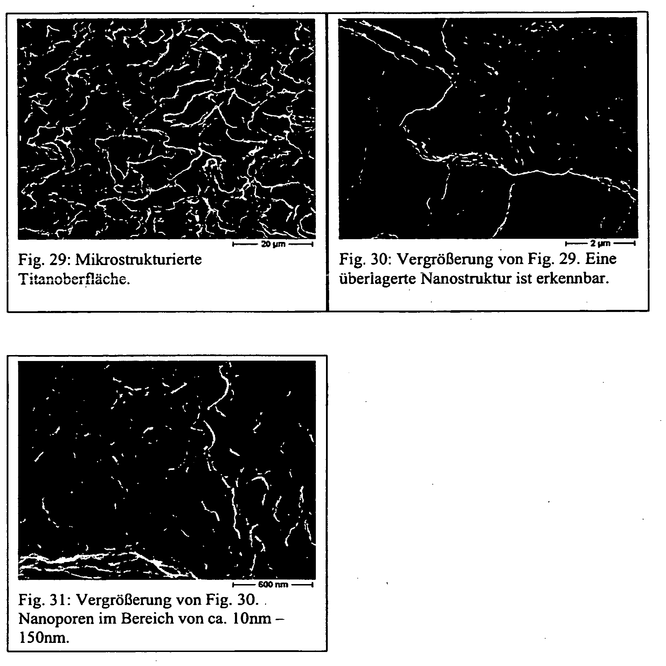

Die Titanoberflächen der Fig. 29 bis 37 wurden bei der Herstellung zuerst der Vorbehandlungsmethode zwei unterzogen um die Mikrostruktur zu erzeugen. Die Nanostrukturen wurden mit mittels der gepulsten Elektrolyse hergestellt. Alle drei Oberflächenvarianten zeigten noch mehrere Wochen nach Lagerung im an normaler Luft einen Benetzungswinkel kleiner 15°. Bedingt durch die Mikrostrukturierung "saugte" sich die Oberfläche auch Wochen nach der Herstellung und Lagerung an normaler Luft bei Kontakt mit Wasser mit diesem voll.

### Bezugszeichenliste

- 2: Pfostenteil
- 4: Aufbauteil
- 6, 7, 8, 9: Stufe
- 10, 11, 12: Gewinde
- 14: apikales Ende
- 15: koronales Ende
- 16: Bohrung
- 18: Innengewinde
- 20: Durchgangsbohrung
- 22: Krone
- 24: Implantat-Grundkörper
- 26: Behälter
- 28, 30: Elektrode
- 32: Draht
- 34: Halteelement

## Patentansprüche

1. Verfahren zur Herstellung eines Metallkörpers mit einer nanoskopische Poren oder eine nanoskopische Struktur aufweisenden Oberfläche, bei dem ein aus Titan oder aus einer titanhaltigen Legierung bestehender Metall-Grundkörper in einem Elektrolysebad pulsierend mit einem Strom beaufschlagt wird, wobei das Elektrolysebad mit Ionen versetzt ist, die jeweils aus einem Element aus einer der V. bis VII. Hauptgruppe des Periodensystems der Elemente bestehen oder ein derartiges Element als Bestandteil umfassen, und bei dem der Metall-Gründkorper mit einem Wechselstrom oder mit einem pulsierenden Gleichstrom beaufschlagt wird, wobei die Amplitude der Steuerspannung des Stroms in aufeinanderfolgenden Zeitintervallen schrittweise gesteigert wird, und die im Transientenbereich eine Änderungsgeschwindigkeit von mindestens 1 V/s aufweist.

2. Verfahren nach Anspruch 1, bei dem die zur Erzeugung des Stroms angelegte Steuerspannung im Transientenbereich eine Änderungsgeschwindigkeit von mindestens 10 V/s, vorzugsweise von 100 V/s aufweist.

3. Verfahren nach Anspruch 1 oder 2, bei dem der Metall-Grundkörper mit Chrom versetzt ist.

4. Verfahren nach einem der Ansprüche 1 bis 3, bei dem die Amplitude in aufeinanderfolgenden Zeitintervallen in Schritten von 5 V oder weniger, von 5 V bis auf 15 V gesteigert wird.

5. Verfahren nach einem der vorhergehenden Ansprüche, bei dem als Steuerspannung ein pulsierendes rechteckförmiges Spannungssignal angelegt wird.

6. Verfahren nach einem der vorhergehenden Ansprüche, bei dem als Elektrolyt eine wässrige Natriumsulfatlösung oder eine Ammoniumsulfatlösung oder eine Natriumnitritlösung oder eine Ammoniumnitritlösung gewählt wird.

7. Verfahren nach einem der Ansprüche 1 bis 6, bei dem ein Dentalimplantat hergestellt wird.

8. Verfahren nach Anspruch 7, bei dem als Metall-Grundkörper ein mit einer mikrostrukturierten Oberfläche versehener Implantat-Grundkörper verwendet wird.

9. Knochenimplantat, gebildet aus Titan oder aus einer titanhaitigen Legierung, mit einer nanoskopische Poren oder eine nanoskopische Struktur aufweisenden Oberfläche, hergestellt durch ein Verfahren nach einem der Ansprüche 1 bis 8, bei dem die nanoskopischen Poren einen mittleren Porendurchmesser von nicht mehr als 250 nm aufweisen bzw. die nanoskopische Struktur eine Strukturgröße von nicht mehr als 250 nm aufweist.

10. Knochenimplantat nach Anspruch 9, dessen Oberfläche einen hydrophilen Benetzungswinkel von nicht mehr als 15° aufweist.

11. Knochenimplantat nach Anspruch 9 oder 10 mit einer mikrostrukturierten Oberfläche, wobei der Mikrostruktur eine Nanostruktur überlagert ist, und wobei im Bereich der Oberfläche Stickstoffatome und/oder Stickstoffverbindungen angelagert und/oder eingeschlossen sind.

## Claims

1. A process for producing a metal body with a surface having nanoscale pores or a nanoscale structure, wherein a pulsating current is applied to a metal base body consisting of titanium or of an alloy containing titanium in an electrolysis bath, with ions being added to the electrolysis bath, which in each case consists of an element of one of main groups V to VII of the periodic system of elements or comprise such an element as a component, and wherein an alternating current or a pulsating direct current is applied to the metal base body, the amplitude of the control voltage of the current being incrementally increased in successive time intervals and having in the transient range a changing speed of at least 1 V/s.

2. The process of claim 1, wherein the control voltage applied for generating the current has in the transient range a changing speed of at least 10 V/s, preferably of 100 V/s.

3. The process of claim 1 or 2, wherein chromium is added to the metal base body.

4. The process of any of claims 1 to 3, wherein the amplitude is increased in successive time intervals in steps of 5 V or less, from 5 V up to 15 V.

5. The process of any of the preceding claims, wherein a pulsating rectangular voltage signal is applied as control voltage.

6. The process of any of the preceding claims, wherein an aqueous sodium-sulphate solution or an ammonium-sulphate solution or an ammonium-nitrite solution is chosen as electrolyte.

7. The process of any of claims 1 to 6, in which a dental implant is produced.

8. The process of claim 7, wherein an implant base body having a microstructured surface is used as metal base body.

9. A bone implant, made from titanium or from an alloy containing titanium, with a surface having nanoscale pores or a nanoscale structure, produced by means of a process of any of claims 1 to 8, wherein the nanoscale pores have a mean pore diameter of not more than 250 nm or the nanoscale structure has a structure size of not more than 250 nm.

10. The bone implant of claim 9, whose surface has an hydrophile wetting angle of not more than 15°.

11. The bone implant of claim 9 or 10 with a microstructured surface, wherein a nanostructure is superimposed on the microstructure and wherein nitrogen atoms and/or nitrogen compounds are attached and/or included in the region of the surface.

## Revendications

1. Procédé de fabrication d'un corps métallique avec une surface ayant des pores nanoscopiques ou une structure nanoscopique, dans lequel un corps de base métallique se composant de titane ou d'un alliage contenant titane est soumis à un courant pulsatif dans un bain d'électrolyse, le bain d'électrolyse étant chargé d'ions qui à chaque fois se composent d'un élément de l'un des groupes principaux V à VII du système périodique des éléments ou comprennent un tel élément en tant que composant, et dans lequel le corps de base métallique est soumis à un courant alternatif ou à un courant continu pulsatif, l'amplitude de la tension de contrôle du courant étant augmentée progressivement dans des intervalles de temps successifs et ayant dans le domaine transitoire une vitesse de changement d'au moins 1 V/s.

2. Procédé selon la revendication 1, dans lequel la tension de contrôle appliquée pour générer le courant a dans le domaine transitoire une vitesse de changement d'au moins 10 V/s, de préférence de 100 V/s.

3. Procédé selon la revendication 1 ou 2, dans lequel du chrome est mélangé au corps de base métallique.

4. Procédé selon l'une quelconque des revendications 1 à 3, dans lequel l'amplitude est augmentée dans des intervalles de temps successifs dans des pas de 5 V ou moins, de 5 V jusqu'à 15 V.

5. Procédé selon l'une quelconque des revendications précédentes, dans lequel un signal de tension rectangulaire pulsatif est appliqué comme tension de contrôle.

6. Procédé selon l'une quelconque des revendications précédentes, dans lequel une solution aqueuse de sulfate de sodium solution ou une solution de sulfate d'ammonium ou une solution de nitrite d'ammonium est choisie comme électrolyte.

7. Procédé selon l'une quelconque des revendications 1 à 6, dans lequel un implant dentaire est fabriqué.

8. Procédé selon la revendication 7, dans lequel un corps de base d'implant ayant une surface microstructurée est utilisé comme corps de base métallique.

9. Implant osseux, fait de titane ou d'un alliage contenant titane, avec une surface ayant des pores nanoscopiques ou une structure nanoscopique, fabriqué par un procédé de l'une quelconque des revendications 1 à 8, dans lequel les pores nanoscopiques ont un diamètre de pore moyen de non plus de 250 nm ou la structure nanoscopique a une taille de structure de non plus de 250 nm.

10. Implant osseux selon la revendication 9, dont la surface a un angle de mouillage hydrophile de non plus de 15°.

11. Implant osseux selon la revendication 9 ou 10 avec une surface microstructurée, dans lequel la microstructure est recouverte d'une nanostructure et dans lequel des atomes d'azote et/ou des composés d'azote sont fixés et/ou inclus au niveau de la surface.
